# EUROPÄISCHE PATENTSCHRIFT

(11) **EP 0 591 152 B1**
(45) Veröffentlichungstag und Bekanntmachung des Hinweises auf die Patenterteilung: **20.03.1996**
(21) Anmeldenummer: 90917193.6
(22) Anmeldetag: 02.11.1990
(51) Int. Cl.: A61M 5/00, A61M 25/00

(54) **INFEKTVORBEUGENDE KATHETERANORDNUNG**
ANTI-INFECTION CATHETER ARRANGEMENT
AGENCEMENT DE CATHETER ANTI-INFECTION

(43) Veröffentlichungstag der Anmeldung: 13.04.1994
(73) Patentinhaber: GIEBEL, Marion, D-42853 Remscheid (DE); PALM, Ülo-Aarne, D-81677 München (DE); BOEMKE, Willehad, D-10707 Berlin (DE)
(72) Erfinder: GIEBEL, Marion, D-42853 Remscheid (DE); PALM, Ülo-Aarne, D-81677 München (DE); BOEMKE, Willehad, D-10707 Berlin (DE)
(86) Internationale Anmeldenummer: DE9000937
(87) Internationale Veröffentlichungsnummer: WO9106329

(56) Entgegenhaltungen:
- US-A- 3 861 388
- US-A- 4 581 014
- US-A- 4 642 091

## Beschreibung

Die Erfindung betrifft eine infektvorbeugende Katheteranordnung der im Oberbegriff des Anspruchs 1 angegebenen Art.

Eine bedeutende und oft lebensgefährliche Komplikation beim Einsatz intravasaler Katheter besteht im Auftreten katheterbedingter Infektionen. Die Infektionsrate nimmt zu, je länger der Katheter im Körper verbleibt. Stellvertretend für eine große Anzahl ähnlicher Untersuchungen sei hier zur Verdeutlichung der Brisanz des Problems nur auf eine im "American Journal of Medicine" 1981 erschienene Studie verwiesen, nach der in den USA jedes Jahr etwa 20 000 Todesfälle auf katheterbedingte Infektionen zurückzuführen sind. Dementsprechend zahlreich und vielfältig sind auch die weltweit zu dieser Problematik durchgeführten und die noch laufenden Forschungsprogramme.

Bekannt ist, daß in den Katheter eingedrungene pathogene Keime dort ideale Wachstumsbedingungen vorfinden und sich bei völligem Fehlen von im Körper vorhandenen Abwehrstoffen bzw. -mechanismen ungehemmt vermehren können. Die Folge ist eine Einschwemmung ganzer Keimkolonien in den Körper des Patienten und damit eine Bakteriämie bzw. Sepsis.

Die bisherigen Versuche, die Infektionsrate zu senken, basieren im wesentlichen auf vier verschiedenen Ansätzen, die darauf abzielen, erstens die Keime abzutöten, zweitens den Keimen den Zutritt in das Innere des Katheters zu verwehren, drittens, spezielle, die Keimanlagerung erschwerende Kathetermaterialien einzusetzen und viertens Kombinationen dieser drei Methoden.

Zu dem ersten Lösungsansatz sind vor allem vier US-Patentschriften bemerkenswert.

In der US-A 4 623 329 wird ein Katheter beschrieben, dessen Kanülenwandung mit keimtötende Wirkstoffe enthaltenden Kapillaren versehen ist. Die Wirkstoffe gelangen durch Diffusion in das Innere des Katheters. Der auf diese Weise erzielbare Effekt ist jedoch aufgrund der zu geringen Wirkstoffkonzentration sehr begrenzt. Nachteilig ist darüberhinaus die ungleichmäßige Freisetzung der Wirkstoffe sowie die komplizierte Konstruktion dieses speziellen Katheters.

Weiterhin sind aus den US-A 4 432 764, 4 440 207 und 4 624 664 technische Lösungen für Verschlußkappensysteme mit einem Wirkstoffreservoir bzw. wirkstoffabsorbierendem Wandmaterial bekannt, die bei Nichtbenutzung des Katheters an dessen rückwärtigem Anschlußstück ansetzbar sind. Derartige Verschlußkappen bekämpfen jedoch nur die bei Entfernung des Infusionsschlauches eventuell auftretende Kontaminierung des Katheterverschlusses und nicht die durch die Kanülenspitze eindringenden Keime. Es ist bekannt, daß sich aber gerade an der Kanülenspitze verstärkt Keime festsetzen.

Der zweite Lösungsansatz beruht auf der Zielstellung, den Keimen den Zutritt in das Innere des Kanülenrohres zu verwehren. Aus der DE-A 35 04 661 ist ein Entenschnabelventil bekannt, das als Einwegventil den Durchtritt der zu injizierenden Medikamente bzw. Lösungen in die Gefäße zuläßt, andererseits jedoch verhindert, daß Flüssigkeiten aus den Gefäßen in den Katheter zurückfließen können. Einwegventile weisen jedoch prinzipiell den Nachteil auf, daß die Überprüfung der Lage des Katheters in einem Blutgefäß durch Ansaugen von Blut nicht möglich ist.

Zum Verschließen des Kanülenrohres bei intermittierender Injektion ist weiterhin das Einschieben eines Mandrins in das Kanülenrohr gebräuchlich. In der DE-A 37 21 299 wird eine Kathetervorrichtung mit einem Mandrin beschrieben, bei der das durch einen Zuspritzport injizierte Medikament durch einen Flüssigkeitskanal zwischen Mandrin und Kanülenrohr fließt und durch ein Ventil aus dem Kanülenrohr austritt. Dadurch, daß der Mandrin für eine Injektion nicht mehr aus dem Kanülenrohr herausgenommen werden muß, wird eine Kontamination des Kanülenlumens weitgehend vermieden.

Gemäß den Vorschlägen beider vorstehend genannter DE-Druckschriften kommen Ventile zum Einsatz, die zwar das Eindringen von Körperflüssigkeit, nicht aber von Keimen verhindern. Mangelnde Dichtheit und vor allem die Öffnung des Ventils während der Injektion führen dazu, daß doch einige Keime in das Kanülenrohr gelangen. Um eine Infektionsgefahr zu verursachen, genügen aber bereits wenige Keime.

Eine dritte Möglichkeit zur Verringerung der Infektionsgefahr besteht darin, für das Kanülenrohr des Katheters keimabwehrendes Material zu verwenden bzw. das Kanülenrohr zumindest stellenweise mit einem derartigen Material zu beschichten.

Eine bakterienabwehrende Mixtur aus biokompatiblem Grundmaterial und antibiotischen Metallen ist in der US-A 4 677 143 angegeben. In der US-A 4 642 104 wird vorgeschlagen, eine antimikrobische Substanz chemisch an ein Basispolymer zu binden. In der klinischen Praxis hat sich jedoch gezeigt, daß mehr Gewebeschädigungen, insbesondere Reizungen und Entzündungen durch diese Katheter als durch unbehandelte Katheter hervorgerufen werden. Zusätzlich ist die Gefahr einer Resistenzentwicklung gegeben.

Eine vierte Herangehensweise an das Problem beruht auf einer Kombination der oben diskutierten ersten drei Lösungsansätze.

In der DE-A 37 47 548 und der US-A 4 676 782 werden Katheter bzw. Zusatzeinheiten für Katheter beschrieben, die darauf abzielen, Mikrobien den Zutritt durch die Implantationswunde zu verwehren, indem antibiotikahaltige Materialien lokal eingesetzt werden. Zu diesem Zweck ist entsprechend der DE-A 37 47 548 die äußere Oberfläche des im Bereich der Körperöffnung befindlichen Kanülenabschnittes mit einer hydrophoben, Mikrobien absorbierenden Beschichtung versehen. Auf einem ähnlichen Prinzip basiert die Wirkung der in der US-A 4 676 782 beschriebenen Zusatzeinheit für beliebige handelsübliche Katheter. Vorgeschlagen wird eine separate Hülse zur Ummantelung des Einstichbereiches. Diese auch unter der Bezeichnung VITA CUFF bekannte Einrichtung hat jedoch ebenso wie die in der DE-A beschriebene Variante nur lokale Wirkung und bringt daher auch nur einen Teilerfolg.

Alle bisher bekannten, oben diskutierten Maßnahmen zur Verringerung und Verhütung katheterbedingter Infektionen führten bisher nur zu sehr geringen Erfolgen.

Auch das Injizieren einer Antibiotikalösung in den Katheter, wobei das injizierte Volumen das Kathetervolumen weit übersteigt (Messing B, Peitra C, Debure A, Beliah M, Bernier J. Antibiotic-lock technique: a new approach to optimal therapy for catheter-related sepsis in home-parenteral nutrition patients. J Parenter Enteral Nutr 1988; 12:185-189) ist nachteilig. Aufgrund des Eindringens der überschüssigen Antibiotikalösung in den Organismus muß die Konzentration des Antibiotikums zwangsläufig so gering gehalten werden, daß sich keine toxischen Nebenwirkungen und Keimresistenzen einstellen können. Dies hat zur Folge, daß die keimtötende Wirkung der zwischen zwei Injektionen im Katheter verbleibenden Antibiotikalösung nicht ausreicht, um alle an der Innenwand des Katheters festhaftenden Keime vollständig abzutöten.

Es besteht deshalb die Aufgabe, eine Katheteranordnung anzugeben, bei der die katheterbedingte Infektionsrate drastisch reduziert ist. Dabei ist im Hinblick auf den Routinebetrieb eine einfache und bequeme Handhabbarkeit anzustreben.

Diese Aufgabe wird mit den kennzeichnenden Merkmalen des Anspruchs 1 gelöst.

Die Erfindung stützt sich auf die Ergebnisse umfangreicher tierexperimenteller Forschungen (Palm Ü, Boemke W, Reinhardt H W, Verhinderung katheterbedingter Infektionen, eine experimentelle Langzeituntersuchung. Anaesthesist 1989;38 Supplement 1:129). Es wurde gefunden, daß bei Hunden die katheterbedingte Infektionsrate von fast 100% auf nahe 0% absenkbar ist, wenn das Kathetervolumen intermittierend mit einer hochkonzentrierten Gentamycinlösung, der etwas Alpha-Chymotrypsin beigemischt ist, gefüllt wird.

Erfindungsgemäß ist eine an das rückwärtige Anschlußstück des Katheters ansetzbare spezielle Vorrichtung dazu vorgesehen, das Kathetervolumen mit mindestens einer antibiotisch wirkenden Substanz mindestens minimaler Wirkkonzentration aufzufüllen und mindestens das gleiche Volumen wieder abzuziehen. Das Füllvolumen stimmt dabei exakt mit dem Kathetervolumen überein.

Durch eine möglichst hohe Konzentration des Antibiotikums ist eine weitere Optimierung der beabsichtigten Wirkung erzielbar. Die antibiotische Wirksubstanz kann zusätzlich mit eiweißspaltenden Enzymen, insbesondere Protease, insbesondere Alpha-Chymotrypsin angereichert sein. Die beispielsweise während des Injektionsvorganges in den Katheter eingedrungenen pathogenen Keime umgeben sich während der Koloniebildung mit einer Eiweißhülle, wodurch die Antibiotika teilweise von den Keimen abgeschirmt werden können, was deren Wirkungseffizienz beeinträchtigt. Es ist anzunehmen, daß eiweißspaltende Enzyme derartige Schutzhüllen zerstören, so daß das Antibiotikum wieder voll zur Wirkung kommen kann.

Auch die Zugabe von Antimykotika zur Bekämpfung pathogener Pilze und von Antikoagulanzien, insbesondere von Heparin zur Verhinderung von Blutgerinnseln und dadurch verursachten Kanülenverstopfungen kann bei bestimmten Katheteranwendungen vorteilhaft sein.

Wichtig ist, daß die Substanz nur innerhalb des Katheters wirkt und nicht in den Körper des Patienten gelangt. Damit ist gewährleistet, daß die höchstmögliche Wirkstoffkonzentration bei Vermeidung der sonst auftretenden Nebenwirkungen und Resistenzentwicklungen zur Anwendung kommen kann. Eine diesbezügliche Einfüll- und Absaugvorrichtung ist entsprechend einer bevorzugten Ausführungsform der Erfindung dadurch gekennzeichnet, daß das abgesaugte Volumen das Kathetervolumen etwas übertrifft, während das Einfüllvolumen exakt dem Kathetervolumen entspricht. Auf diese Weise ist sichergestellt, daß keine Wirkstoffreste im Katheter zurückbleiben und in den Körper des Patienten eindringen können.

Gemäß einer besonders bevorzugten Ausführungsform der Erfindung ist die Vorrichtung mit zwei getrennten Substanzreservoiren für Alpha-Chymotrypsin und Gentamycin ausgestattet. Eine derartige Vorrichtung weist vorzugsweise zylindrische Form auf, wobei die Substanzreservoire axial hintereinander angeordnet sind. Das katheterseitig erste Reservoir enthält das Alpha-Chymotrypsin. Beide Reservoire sind lediglich durch eine dünne Membran getrennt, welche zum Zwecke des Vermischens der beiden Substanzen leicht zerstörbar ist. Die Vermischung des pulverförmigen Alpha-Chymotrypsin mit dem flüssigen Gentamycin darf jedoch erst unmittelbar vor oder während des Einfüllvorganges in den Katheter erfolgen, da das aufgelöste Chymotrypsin seine Wirkung nur während eines kurzen Zeitraumes entfaltet.

Das Anschlußstück der Einfüll- und Absaugvorrichtung ist dem des Katheters angepaßt, so daß eine dichtende Verbindung gewährleistet ist. Da sich für Katheteranschlüsse vor allem Luer-Gewinde durchgesetzt haben, ist jedoch eine weitgehende Vereinheitlichung möglich.

Das zweikammrige Wirkstoffreservoir der Vorrichtung ist durch eine zweite Membran gegenüber der Katheteröffnung verschlossen. Diese Membran reißt durch den Druck eines innerhalb des zylinderförmigen Reservoirs verschieblichen Stempels, wodurch gleichzeitig die Gentamycin/Chymotrypsin-Lösung in den Katheter gedrückt wird. Die Stempelbewegung ist besonders einfach und sicher mittels einer mit dem Stempel verbundenen, auf die äußere Wandung der Vorrichtung aufschraubbaren Kappe erzeugbar. Auf die gleiche Weise ist der Stempel zum Ansaugen der im Katheter befindlichen Wirksubstanz in der entgegengesetzten Richtung verschiebbar, wobei der Rückstellweg etwas über die Ausgangsposition hinausgeht. Damit ist garantiert, daß kein kleinster Rest des hochkonzentrierten Antibiotikums in den Körper des Patienten eingespült werden kann.

Um gegebenenfalls die in den Vorrichtungszylinder zurückgesaugte Flüssigkeit im keimabgeschirmten Zustand einem Analyselabor zuzuführen, ist eine sterile, separat verpackte Verschlußkappe vorgesehen. Derartige Analysen dienen der Kontrolle der Keimfreiheit. Beispielsweise können in den gegen Bakterien geschützten Katheter Pilze gefunden werden, wodurch der Einsatz einer ein Antimykotikum enthaltenden Vorrichtung erforderlich ist. Die Vorrichtung ist vorzugsweise zum einmaligen Gebrauch bestimmt. Bei Einkammersystemen, die statt einer Membran ein für den Durchlaß in beiden Richtungen geeignetes Ventil aufweisen, ist jedoch eine Wiederverwendbarkeit denkbar. Voraussetzung dafür ist weiterhin eine Säuberung und Sterilisation zwischen zwei Anwendungen und die Auffüllung mit neuer Wirksubstanz, wobei insbesondere der Einschluß von Luftblasen zu vermeiden ist. Ein solcher Aufwand ist jedoch in Anbetracht des einfachen Aufbaus einer einkammrigen Einfüll- und Ansaugvorrichtung schon aus wirtschaftlichen Gründen kaum gerechtfertigt.

Die Vorrichtung besteht vorzugsweise zumindest in ihren wesentlichen Teilen aus Kunststoff. Durchsichtiger Kunststoff ist vor allem für die Kammer(n) vorteilhaft, wenn eine der Stempelverschiebung entsprechende Skalierung vorgesehen ist. Im allgemeinen ist das Kathetervolumen zwar konstant, so daß das Volumen des Wirkstoffreservoirs bereits auf das Kathetervolumen abgestimmt ist, wodurch sich eine Skalierung erübrigt. Im Einzelfall kann es jedoch erforderlich sein, den Kanülenschlauch wegen Versprödungserscheinungen oder mechanischer Beschädigung zu kürzen. In diesem Fall kann das verringerte Kathetervolumen durch eine Skalierung des Einfüllvolumens ausgeglichen werden.

Entsprechend einer Weiterbildung der Erfindung ist zwischen dem Katheter und der Vorrichtung ein Dreiwegehahn angeordnet. Dieser erlaubt den Anschluß von zwei Zuleitungen an das Katheteranschlußstück bei Freigabe einer Durchlaßrichtung oder Verschluß beider Richtungen. Das zu füllende Volumen setzt sich dann aus dem Katheterfüllvolumen und dem Innenvolumen des Dreiwegehahns zusammen. Vorteilhaft ist bei dieser Anordnung vor allem die Tatsache, daß das alternative Anschließen des Infusionsschlauches und der Einfüll- und Ansaugvorrichtungen entfällt. Beide Systeme sind ständig mit dem Katheteranschlußstück verbunden. Der Austausch der beiden Systeme reduziert sich auf ein Umschalten des Dreiwegehahnes. Damit ist eine Zeiteinsparung sowie eine weitere Erhöhung der Kontaminationssicherheit verbunden. Das Eindringen von Gentamycin in den Kreislauf sollte wegen möglicher Nebenwirkungen - besonders gefürchtet sind die Nephro- und Ototoxizität - und Resistenzentwicklungen vermieden werden. Als eine vorteilhafte Weiterbildung der Erfindung wurde deshalb an der Kanülenspitze ein Ventil angeordnet. Vorzugsweise ist das Ventil als Lamelle ausgebildet, die sich durch Injektionsdruck oder Saugwirkung pendeltürartig bewegt. Durch die vorstehend beschriebene Katheteranordnung läßt sich die Gefahr katheterbedingter Infektionen bei Mensch und Tier drastisch reduzieren bzw. endgültig beseitigen. Die einfache Handhabbarkeit der Einfüll- und Ansaugvorrichtung gestattet den routinemäßigen Einsatz und darüberhinaus die Bedienung durch medizinisches Hilfspersonal oder sogar durch den Patienten selbst.

Die Erfindung wird nachfolgend anhand bevorzugter Ausführungsbeispiele erläutert. Es zeigen:
Figur 1a, 1b, 1c, 1d vier Anwendungsphasen einer ersten Variante einer Einfüll- und Ansaugvorrichtung in Schnittdarstellungen,
Figur 2 eine zweite Variante einer Einfüll- und Ansaugvorrichtung in Schnittdarstellung,
Figur 3 eine Anwendungssituation mit einem Dreiwegehahn und
Figur 4 eine perspektivische Darstellung eines Kanülenverschlusses.

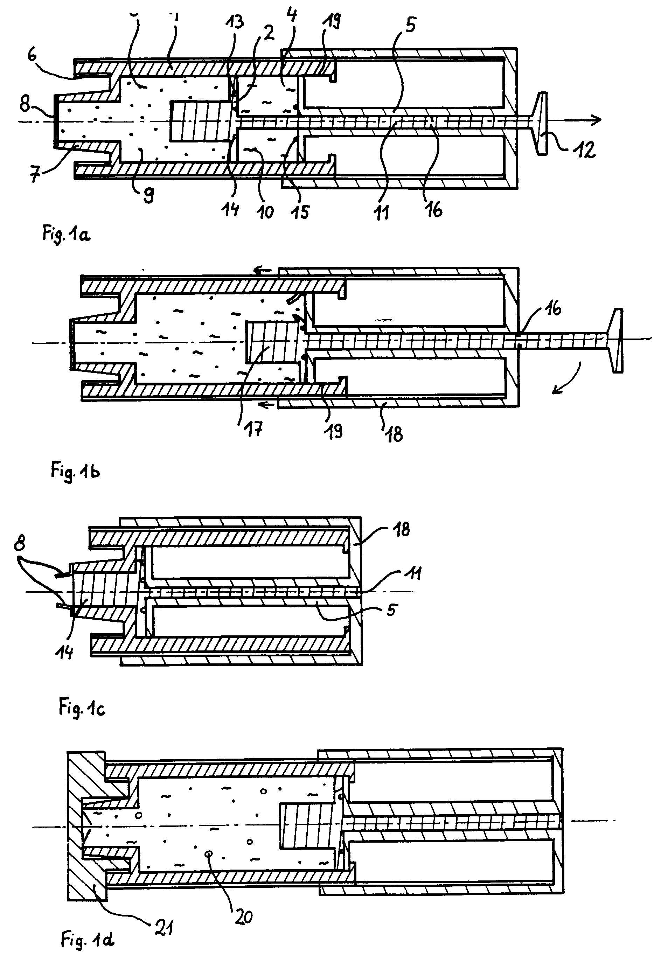

Die in Figur 1a dargestellte Einfüll- und Ansaugvorrichtung besteht im wesentlichen aus einem zylindrischen Gehäuse 1, einer beweglichen Wand 2, die den Innenraum in zwei axial hintereinanderliegende Kammern 3 und 4 teilt sowie einem Stempel 5, welcher die katheterabgewandte stirnseitige Begrenzung der Vorrichtung bildet. Zum Anschluß an den Katheter dienen ein Luer-Gewinde 6 und ein Steckkonus 7. Der Steckkonus 7 ist an seiner Spitze mit einer Membran 8 verschlossen. Die von der Membran 8, dem Steckkonus 7, einem Teil des zylindrischen Gehäuses 1 und der beweglichen Wand 2 begrenzte erste Kammer, ist mit Alpha-Chymotrypsin 9 gefüllt, während die von der beweglichen Wand 2, einem weiteren Teil des Gehäuses 1 und der Frontfläche des Stempels 5 begrenzte zweite Kammer 4 das Gentamycin 10 enthält. Dieses Zweikammersystem bildet eine geschlossene Einheit, die völlig steril in einer nicht dargestellten entsprechenden Verpackungseinheit mit einer ebenfalls nicht dargestellten Schutzkappe für die Membran 8 vorliegt.

Die Schutzkappe, welche an dem Luer-Gewinde 6 der Vorrichtung befestigt ist, wird unmittelbar vor dem Anschluß an den Katheter entfernt, damit eine Kontamination des Anchlußstückes, insbesondere der Membran 8 durch in der Luft ständig vorhandene Keime weitestgehend vermieden wird. Zum Vermischen der beiden Substanzen laßt sich die Wand 2 durch eine Zugstange 11, die axial durch den Stempelkörper 5 hindurchgeführt und mit einem Griff 12 versehen ist, anziehen, wodurch eine vorgeprägte Sollbruchstelle 13 der Wand 2 einreißt. Im vollständig zurückgezogenen Zustand rastet eine Ringnut 14 an der Rückseite der Wand 2 in eine entsprechende Ringwulst 15 an der Stempelfrontfläche ein. Die Zugstange 11 ist mit einer Sollbruchstelle 16 versehen, wodurch das Griffende nach dem Vermischungsvorgang abbrechbar ist. Die zurückgezogene Position ist in der Figur 1b dargestellt. Die nunmehr in dem gesamten Reservoir enthaltene Alpha Chymotrypsin/Gentamycin-Lösung läßt sich durch den Stempel 5 in den Katheter hineindrücken. Die Frontfläche des Stempels 5 wird von der beweglichen Wand 2 gebildet. Die Wand 2 ist deshalb mit einer Ausformung 17 versehen, die dem Innenraum des Steckkonus angepaßt ist. Eine derartige Ausformung 17 ist für die Funktionsfähigkeit der Vorrichtung zwar nicht unbedingt erforderlich, bei Wegfall der Ausformung 17 ist allerdings das Gesamtvolumen der beiden Kammern 3 und 4 um das Volumen des Konusinnenraumes zu erhöhen. Der Stempel 5 ist mittels einer Schraubkappe 18 in den Zylinder eindrehbar. Die Schraubkappe 18 kann auch als separate Überwurfkappe ausgeführt sein, wodurch eine translatorische Stempelbewegung bewirkt wird. Figur 1c zeigt die Vorrichtung nach dem Einfüllen der Wirksubstanz in den Katheter. Die Membran 8 ist durch den Stempeldruck zerstört worden. Die Länge der Vorrichtung beträgt bei üblichen Kathetergrößen maximal einige cm und der Durchmesser einige mm, so daß die zusätzliche Belastung für den Patienten minimal ist. Durch das Zurückschrauben der Kappe 18 läßt sich die Antibiotikalösung in das Reservoir der Vorrichtung zurücksaugen. Der Rückschraubweg ist um die in den Figuren 1a und 1b mit 19 bezeichnete Passungslänge gegenüber dem Einschraubweg verlängert. Folglich wird etwas Körperflüssigkeit aus dem katheterisierten Hohlraum, insbesondere Blut 20 (Figur 1d), über das reine Füllungsvolumen hinaus angesaugt. Dadurch ist sichergestellt, daß keine Wirkstoffrückstände im Katheter verbleiben, die durch eine nachfolgende Infusion in den Körper des Patienten eingespült werden könnten. Nach dem Ansaugvorgang kann die Vorrichtung noch zur Überführung der angesaugten Flüssigkeit in ein Labor dienen, um dort beispielsweise auf ihren Keimgehalt hin untersucht zu werden. Dazu wird die Einfüll- und Ansaugvorrichtung unmittelbar nach der Dekonnektion vom Katheter, wie in der Figur 1d ersichtlich, mit einer sterilen Kappe 21 verschlossen.

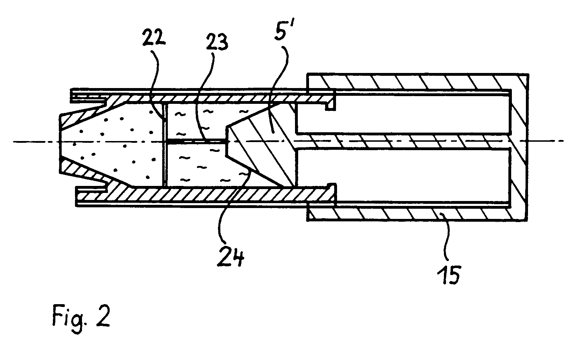

Figur 2 zeigt eine zweite Variante einer 2-kammrigen Einfüll- und Ansaugvorrichtung. Die bewegliche Wand 2 der ersten Variante ist hier zu einer dünnen Membran 22 umgebildet, welche von einer mit dem Stempel 5' mitbewegten Nadel 23 zerstört wird. Die Zylinderform des katheterseitigen Reservoirs ist mit einer kegeligen Spitze versehen, was zur Folge hat, daß die zylinderstumpfartige Ausformung 17 der beweglichen Wand 2 durch eine kegelstumpfartige Ausformung 24 des Stempels 5' ersetzt ist.

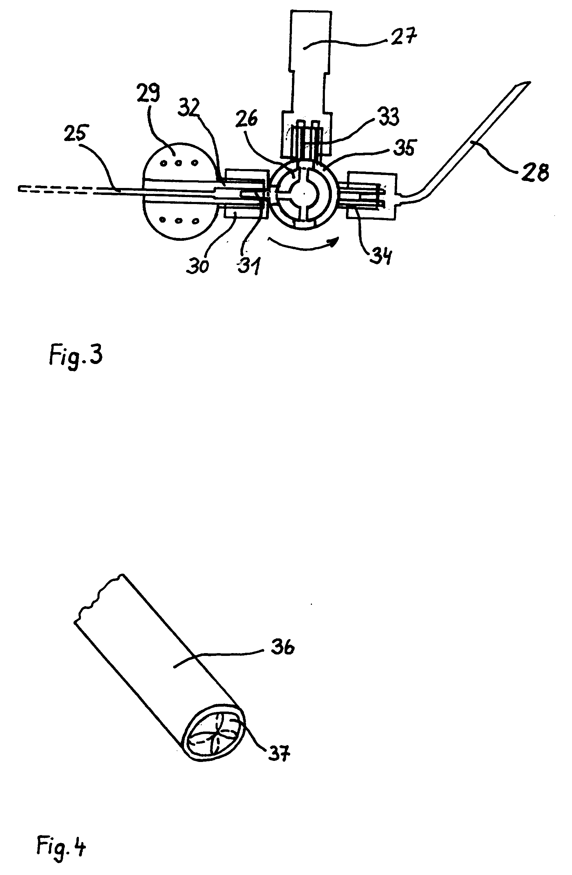

Figur 3 zeigt ein Ausführungsbeispiel einer infektvorbeugenden Katheteranordnung mit einem Katheter 25, einem Dreiwegehahn 26, einer Einfüll- und Ansaugvorrichtung 27 und einem Infusionsschlauch 28, der zu einem nicht dargestellten Behälter für die Infusionsflüssigkeit führt. Der Katheter 25 ist an seinem anschlußseitigen Ende beidseitig mit Flügeln 29 versehen, die durch Heftpflaster an der Haut fixiert werden können. Der Dreiwegehahn 26 weist eine Luer-Überwurfschraubkappe 30 und einen Steckkonus 31 zum Anschluß an den Katheterausgang 32 sowie zwei Luer-Gewinde-Ausgänge 33 und 34 auf. Diese Ausgänge 33 und 34 sind mit einer Einfüll- und Ansaugvorrichtung 27 bzw. dem Infusionsschlauch 28 verbunden. Es ist ersichtlich, daß in der dargestellten Position eine offene Flußrichtung zwischen der Vorrichtung 27 und dem Katheter 25 besteht, während der Infusionsschlauch 28 von dem Katheter 25 getrennt ist. Bei einer Drehung des äußeren Rings 35 des Dreiwegehahns 26 mittels eines nicht dargestellten Griffs um 90 Grad in Pfeilrichtung wird die Durchflußrichtung zwischen dem Infusionsschlauch und dem Katheter 25 geöffnet und die Verbindung zwischen der Einfüll- und Ansaugvorrichtung 27 und dem Katheter 25 geschlossen. Auf diese Weise ist nach Entfernung der Katheterfüllung durch die Einfüll- und Ansaugvorrichtung 27 eine schnelle Umschaltung auf Infusion möglich. Sowohl der Infusionsschlauch 28 als auch die Vorrichtung 27 bleiben mit dem Katheterausgang verbunden, wodurch das Wechseln der Anschlüse entfällt. Das Füllvolumen der Vorrichtung 27 ist um das Innenvolumen des Dreiwegehahnes 26 vergrößert.

In der Figur 4 ist eine Variante für einen ventilartigen Verschluß der Kanülenspitze 36 dargestellt. Mehrere Lamellen 37, die mit der Wandung der Kanülenspitze 36 verbunden sind, überlappen sich irisblendenartig, wodurch bei ausgeglichenen Druckverhältnissen ein stabiler und gut dichten-der Verschluß der Kanülenspitze 36 resultiert. Die Öffnung des Verschlusses ist bei Druck- bzw. Saugeinwirkung in beiden Richtungen gleichermaßen möglich. Durch die gegenseitige Überlappung der Lamellen 37 ist eine federnde Rückstellkraft vorhanden, die ein schnelles und exaktes Schließen auch nach häufigem Öffnen garantiert.

## Patentansprüche

1. Infektvorbeugende Katheteranordnung mit einem Katheter (25), der ein starres oder flexibles Kanülenrohr mit einem am rückwärtigen Ende vorgesehenen Anschlußstück (32) aufweist,
**gekennzeichnet durch**
eine an das Anschlußstück (32) ansetzbare Einfüll- und Ansaugvorrichtung (27) mit einem oder mehreren Wirkstoffreservoir(en) mit einem Gesamtvolumen, das dem Katheterfüllvolumen, gegebenenfalls zuzüglich des Volumens von Zwischenstücken, insbesondere eines Dreiwegehahnes (26), entspricht, wobei dieses Volumen vollständig ausgefüllt ist und mindestens eines der Wirkstoffreservoire mit einer Substanz, enthaltend mindestens ein Antibiotikum bzw. Chemotherapeutikum bzw. antivirales Mittel, vorzugsweise Aminoglycosid, insbesondere Gentamycin (10), in mindestens minimaler Wirkkonzentration, gefüllt ist.

2. Katheteranordnung nach Anspruch 1, **dadurch gekennzeichnet,** daß das Antibiotikum bzw. Chemotherapeutikum bzw. antivirale Mittel die höchstmögliche Konzentration aufweist.

3. Katheteranordnung nach einem der vorangehenden Ansprüche, **dadurch gekennzeichnet,** daß die Substanz mindestens ein eiweißspaltendes Enzym, insbesondere Protease, insbesondere Alpha-Chymotrypsin (8) enthält.

4. Katheteranordnung nach einem der vorangehenden Ansprüche, **dadurch gekennzeichnet,** daß die Substanz mindestens ein Antimykotikum enthält.

5. Katheteranordnung nach einem der vorangehenden Ansprüche, **dadurch gekennzeichnet,** daß die Substanz mindestens ein Antikoagulanz, insbesondere Heparin enthält.

6. Katheteranordnung nach einem der vorangehenden Ansprüche, **dadurch gekennzeichnet,** daß die Einfüll- und Absaugvorrichtung zum Absaugen mindestens des Einfüllvolumens ausgebildet ist.

7. Katheteranordnung nach einem der vorangehenden Ansprüche, **dadurch gekennzeichnet,** daß die Vorrichtung zwei Substanzreservoire (3 und 4) aufweist.

8. Katheteranordnung nach Anspruch 7, **dadurch gekennzeichnet,** daß die Vorrichtung eine im wesentlichen zylindrische Form aufweist, wobei die Substanzreservoire (3 und 4) axial hintereinander angeordnet sind.

9. Katheteranordnung nach Anspruch 8, **dadurch gekennzeichnet,** daß das katheterseitige erste Reservoir (3) ein eiweißspaltendes Enzym, insbesondere Alpha-Chymotrypsin (9) und das zweite Reservoir (4) das Antibiotikum bzw. Chemotherapeutikum bzw. antivirale Mittel, insbesondere Gentamycin (10) enthält.

10. Katheteranordnung Anspruch 9, **dadurch gekennzeichnet,** daß die beiden Reservoire (3 und 4) durch eine Membran (21) getrennt sind.

11. Katheteranordnung nach Anspruch 10, **dadurch gekennzeichnet,** daß das erste Reservoir (3) eine dem Katheteranschlußstück (32) angepaßte, mit einer Membran (8) verschlossene Spitze aufweist.

12. Katheteranordnung nach Anspruch 11, **dadurch gekennzeichnet,** daß ein innerhalb der Reservoire (3 und 4) verschieblicher Stempel (5 bzw. 5') vorgesehen ist, dessen Verschiebeweg mindestens der axialen Länge der beiden Reservoire (3 und 4) entspricht und der mit der Wandung der Reservoire dichtend abschließt.

13. Katheteranordnung nach einem der vorangehenden Ansprüche, **dadurch gekennzeichnet,** daß der Anschluß zwischen der Vorrichtung und dem Katheter als Luer-Verschluß (6) ausgebildet ist.

14. Katheteranordnung nach einem der vorangehenden Ansprüche, **dadurch gekennzeichnet,** daß die Vorrichtung (27) für einmaligen Gebrauch bestimmt ist.

15. Katheteranordnung nach einem der vorangehenden Ansprüche, **dadurch gekennzeichnet,** daß die Vorrichtung (27) im wesentlichen aus Kunststoff, insbesondere durchsichtigem Kunststoff besteht.

16. Katheteranordnung nach einem der vorangehenden Ansprüche, **dadurch gekennzeichnet,** daß die Vorrichtung (27) mit einer der Stempelverschiebung entsprechenden Skalierung versehen ist.

17. Katheteranordnung nach einem der vorangehenden Ansprüche, **dadurch gekennzeichnet,** daß der Katheter (25) im wesentlichen aus einem biokompatiblen Material, insbesondere Polyurethan besteht.

18. Katheteranordnung nach einem der vorangehenden Ansprüche, **dadurch gekennzeichnet,** daß der Katheter (25) ein Verweilkatheter ist.

19. Katheteranordnung nach einem der vorangehenden Ansprüche, **dadurch gekennzeichnet,** daß die Kanülenspitze (36) Mittel zum Verschließen der Kanülenöffnung aufweist.

20. Katheteranordnung nach Anspruch 19, **dadurch gekennzeichnet,** daß durch Injektionsdruck bzw. Saugwirkung in beiden Richtungen pendeltürartig bewegliche Lamellen vorgesehen sind.

21. Katheteranordnung nach einem der vorangehenden Ansprüche, **dadurch gekennzeichnet,** daß die Vorrichtung (27) zum Auffüllen des Kathetervolumens und des Volumens eines zwischen dem Katheter (25) und der Vorrichtung (27) angeordneten Dreiwegehahnes (26) vorgesehen ist.

22. Katheteranordnung nach einem der vorangehenden Ansprüche, **dadurch gekennzeichnet,** daß die Vorrichtung (27) als geschlossenes System ausgebildet ist.

23. Katheteranordnung nach einem der vorangehenden Ansprüche, **dadurch gekennzeichnet,** daß die Vorrichtung eine abnehmbare Schutzkappe aufweist.

24. Katheteranordnung nach einem der vorangehenden Ansprüche, **dadurch gekennzeichnet,** daß für die Vorrichtung (27) eine separate sterile Verpakkungseinheit vorgesehen ist.

25. Katheteranordnung nach einem der vorangehenden Ansprüche, **dadurch gekennzeichnet,** daß eine sterile, separat verpackte Verschlußkappe (21) für die Vorrichtung (27) vorgesehen ist.

## Claims

1. Infection-preventing catheter arrangement with a catheter, which exhibits a rigid or flexible catheter tube with a connection piece furnished at the distal end of the catheter tube,
characterized in that
a filling and suction device (27), connectable to the connection piece 32, with one or several active-agent reservoir(s) with a total volume wherein the total volume corresponds to the catheter filling volume, possibly with the addition of the volume of intermediate pieces, in particular of a three-way valve, wherein this volume is completely filled, and wherein at least one of the active-agent reservoirs is filled with a substance, containing at least one antibiotic or, respectively, chemotherapeutic agents or, respectively, antiviral agent, preferably aminoglycoside, in particular gentamycin 10, in at least a minimum active concentration,

2. Catheter arrangement according to claim 1, characterized in that the antibiotic or, respectively, chemotherapeutic agent or, respectively, the antiviral agent exhibits the highest possible concentration.

3. Catheter arrangement according to one of the preceding claims, characterized in that the substance contains at least one proteolytic enzyme, in particular protease, in particular alpha-chymotrypsin (8).

4. Catheter arrangement according to one of the preceding claims, characterized in that the substance contains at least one antimycotic agent.

5. Catheter arrangement according to one of the preceding claims, characterized in that the substance contains at least one anticoagulant, in particular heparin.

6. Catheter arrangement according to one of the preceding claims, characterized in that the filling and suction device is formed for the suction of at least the fill-in volume.

7. Catheter arrangement according to one of the preceding claims, characterized in that the device exhibits two substance reservoirs (3 and 4).

8. Catheter arrangement according to claim 7, characterized in that the device exhibits essentially a cylindrical shape, wherein the substance reservoirs (3 and 4) are disposed sequentially in axial direction.

9. Catheter arrangement according to claim 8, characterized in that the first reservoir (3) on the catheter side contains an proteolytic enzyme, in particular alpha-chymotrypsin (9) and that the second reservoir (4) contains the antibiotic agent or, respectively, the chemotherapeutic agent or, respectively, the antiviral agent, in particular gentamycin (10).

10. Catheter arrangement according to claim 9, characterized in that the two reservoir (3 and 4) are separated by a membrane 21.

11. Catheter arrangement according to claim 10, characterized in that the first reservoir (3) exhibits a tip closed with a membrane (8), where the tip is adapted to the catheter connection piece (32).

12. Catheter arrangement according to claim 11, characterized in that a plunger (5 or, respectively 5'), shiftable inside the reservoirs (3 and 4), is furnished, where a shifting path of the plunger (5 or, respectively, 5'), corresponds at least to the axial length of the two reservoirs (3 and 4) and wherein the plunger (5 or, respectively, 5') sealingly closes with the wall of the reservoirs.

13. Catheter arrangement according to one of the preceding claims, characterized in that the connection between the device and the catheter is formed as a Luer closure (6).

14. Catheter arrangement according to one of the preceding claims, characterized in that the device (27) is intended for single use.

15. Catheter arrangement according to one of the preceding claims, characterized in that the device (27) comprises essentially plastic, in particular, transparent plastic.

16. Catheter arrangement according to one of the preceding claims, characterized in that the device (27) is furnished with a scaling corresponding to the plunger shifting.

17. Catheter arrangement according to one of the preceding claims, characterized in that the catheter (25) comprises essentially a biocompatible material, in particular polyurethane.

18. Catheter arrangement according to one of the preceding claims, characterized in that the catheter (25) is an indwelling catheter.

19. Catheter arrangement according to one of the preceding claims, characterized in that the catheter tube tip (36) exhibits means for closing the catheter tube opening.

20. Catheter arrangement according to claim 19, characterized in that lamellae are furnished, which are movable like a swinging door in two directions, based on the injection pressure or, respectively, the suction action.

21. Catheter arrangement according to one of the preceding claims, characterized in that the device (27) is furnished for the filling of the catheter volume and of the volume of a three-way valve (26) disposed between the catheter (25) and the device (27).

22. Catheter arrangement according to one of the preceding claims, characterized in that the device (27) is formed as a closed system.

23. Catheter arrangement according to one of the preceding claims, characterized in that the device exhibits a removable protective cap.

24. Catheter arrangement according to one of the preceding claims, characterized in that a separate sterile packaging unit is furnished for the device (27).

25. Catheter arrangement according to one of the preceding claims, characterized in that a sterile, separately packed closure cap (21) is furnished for the device (27).

## Revendications

1. Equipement de cathéter évitant les infections avec un cathéter (25) qui comprend une canule rigide ou flexible munie à son extrémité arrière d'une pièce de raccordement (32)
**caractérisé par**
un dispositif de remplissage et d'aspiration (27) adaptable à la pièce de raccordement (32), avec une ou plusieurs chambre(s) pour agents actifs, dont le volume total correspond au volume de remplissage du cathéter, le cas échéant augmenté du volume de composants intermédiaires, notamment d'un robinet à trois voies (26), ce volume étant complètement rempli, et au moins l'une des chambres contenant une substance avec au moins un antibiotique ou un chimiothérapeutique ou un agent antivireux, de préférence de l'aminoglycoside, notamment de la gentamycine (10), dans une concentration active minimale.

2. Equipement suivant la revendication 1, **caractérisé par le fait que** l'antibiotique ou le chimiothérapeutique ou l'agent antivireux est à la concentration maxi possible.

3. Equipement suivant l'une des revendications précédentes, **caractérisé par le fait que** la substance contient au moins un enzyme protéoclastique, notamment de la protéase, spécialement de l'alpha-chymotrypsine (9).

4. Equipement suivant l'une des revendications précédentes, **caractérisé par le fait que** la substance contient au moins un antimycosique.

5. Equipement suivant l'une des revendications précédentes, **caractérisé par le fait que** la substance contient au moins un anticoagulant, notamment de l'héparine.

6. Equipement suivant l'une des revendications précédentes, **caractérisé par le fait que** le dispositif de remplissage et d'aspiration est conçu pour l'évacuation d'au moins le volume de remplissage.

7. Equipement suivant l'une des revendications précédentes, **caractérisé par le fait que** le dispositif comprend deux chambres pour agents actifs (3 et 4).

8. Equipement suivant la revendication 7, **caractérisé par le fait que** le dispositif présente une forme essentiellement cylindrique, les chambres (3 et 4) étant disposées l'une dans l'axe de l'autre.

9. Equipement suivant la revendication 8, **caractérisé par le fait que** la première chambre (3) du côté du cathéter contient un enzyme protéoclastique, notamment de l'alpha-chymotrypsine (9), et la deuxième chambre (4), l'antibiotique ou le chimiothérapeutique ou l'agent antivireux, notamment de la gentamycine (10).

10. Equipement suivant la revendication 9, **caractérisé par le fait que** les deux chambres (3 et 4) sont séparées entre elles par une membrane (21).

11. Equipement suivant la revendication 10, **caractérisé par le fait que** la première chambre (3) est munie d'une pointe fermée par une membrane (8) et adaptée à la pièce de raccordement (32).

12. Equipement suivant la revendication 11, **caractérisé par le fait** q u'il est prévu dans les chambres (3 et 4) un piston (5 ou 5') dont la course est au moins égale à la longueur des deux chambres (3 et 4) et qui coulisse de façon étanche le long de la paroi des chambres.

13. Equipement suivant l'une des revendications précédentes, **caractérisé par le fait que** le raccordement du dispositif sur le cathéter est réalisé sous forme d'un joint genre baïonnette (6) dit Luer.

14. Equipement suivant l'une des revendications précédentes, **caractérisé par le fait que** le dispositif (27) est prévu pour usage unique.

15. Equipement suivant l'une des revendications précédentes, **caractérisé par le fait que** le dispositif (27) est fait essentiellement en matière plastique, notamment transparente.

16. Equipement suivant l'une des revendications précédentes, **caractérisé par le fait que** le dispositif (27) est muni d'une échelle correspondant aux déplacements du piston.

17. Equipement suivant l'une des revendications précédentes, **caractérisé par le fait que** le cathéter (25) est fait d'une matière biocompatible, notamment de polyuréthanne.

18. Equipement suivant l'une des revendications précédentes, **caractérisé par le fait que** le cathéter (25) est une sonde à demeure.

19. Equipement suivant l'une des revendications précédentes, **caractérisé par le fait que** la pointe de la canule (36) est munie d'organes de fermeture.

20. Equipement suivant la revendication 19, **caractérisé par le fait que** des lamelles sont prévues s'ouvrant dans les deux sens comme une porte battante sous la pression d'injection ou l'effet de succion.

21. Equipement suivant l'une des revendications précédentes, **caractérisé par le fait que** le dispositif (27) est prévu pour le remplissage du volume du cathéter et de celui d'un robinet à trois voies (26) disposé entre le cathéter (25) et le dispositif (27).

22. Equipement suivant l'une des revendications précédentes, **caractérisé par le fait que** le dispositif (27) est conçu comme système fermé.

23. Equipement suivant l'une des revendications précédentes, **caractérisé par le fait que** le dispositif est doté d'un chapeau amovible.

24. Equipement suivant l'une des revendications précédentes, **caractérisé par le fait** qu'il est prévu pour le dispositif (27) un emballage stéril séparé.

25. Equipement suivant l'une des revendications précédentes, **caractérisé par le fait** qu'il est prévu pour le dispositif (27) un couvercle stéril (21) emballé séparément.
